# EUROPEAN PATENT APPLICATION

(11) **EP 3 760 248 A1**
(43) Date of publication of application: **06.01.2021**
(21) Application number: 19775879.0
(22) Date of filing: 28.03.2019
(51) Int. Cl.: A61M 5/142, A61M 5/145, A61M 5/168

(54) **DRUG SOLUTION ADMINISTRATION DEVICE AND CONTROL METHOD OF DRUG SOLUTION ADMINISTRATION DEVICE**

(30) Priority: 30.03.2018 JP 2018067431
(71) Applicant: TERUMO Kabushiki Kaisha, Tokyo 151-0072 (JP)
(72) Inventor: KONDO, Akira, Ashigarakami-gun, Kanagawa 259-0151 (JP); YAKUSHIJI, Yusuke, Ashigarakami-gun, Kanagawa 259-0151 (JP)
(74) Representative: TBK
(86) International application number: PCT/JP2019/013893
(87) International publication number: WO 2019/189703

(57) **Abstract**

There is provided a liquid chemical administration device and a control method for a liquid chemical administration device that can prevent liquid supply of a liquid chemical from ending in a state where the liquid chemical remains in a liquid chemical container.

A liquid chemical administration device 100 has a liquid chemical container 110 filled with a liquid chemical and having a distal end opening at a distal end 112, a housing 120 that holds the liquid chemical container, a pusher 130 that pushes out the liquid chemical in the liquid chemical container, a drive mechanism 140 that advances the pusher toward the distal end opening of the liquid chemical container, a detection unit 150 that detects a detection target part 134 of the pusher and detects liquid supply completion of the liquid chemical based on a detection result, and a control unit 160 that controls an operation of the drive mechanism. The control unit further advances the pusher after the detection unit detects the liquid supply completion.

## Description

### Technical Field

The present invention relates to a liquid chemical administration device and a control method for a liquid chemical administration device.

### Background Art

Conventionally, there is known a syringe pump liquid chemical administration device that administers a liquid chemical filled in a liquid chemical container into a living body by a pushing action of a pusher. This type of liquid chemical administration device includes a cylindrical liquid chemical container and a pusher mechanism that pushes out the liquid chemical in the liquid chemical container (see, for example, Patent Literature 1 below). The liquid chemical container and the pusher mechanism are accommodated in a housing and supported by the housing.

### Citation List

### Patent Literature

Patent Literature 1: JP H9-294807 A

### Summary of Invention

### Technical Problem

In the liquid chemical administration device as described above, completion of a liquid chemical filled in the liquid chemical container is performed, for example, by detecting, with a detection switch installed on an electric board or the like, that the pusher mechanism has moved forward by a predetermined distance. When the detection switch comes into contact with the pusher mechanism and the contact is detected, a control unit mounted on the liquid chemical administration device determines that the liquid chemical has run out, and stops a drive mechanism that moves the pusher forward. After that, the control unit notifies a user that the liquid supply is completed.

In the liquid chemical administration device, if there is a deviation in installation accuracy of the detection switch, the pusher mechanism, the liquid chemical container, and the like, or dimensional accuracy of the pusher mechanism, the liquid chemical container, and the like, it becomes difficult to accurately detect a movement amount of the pusher mechanism by the detection switch. For example, if there is a deviation in the installation accuracy and the dimensional accuracy as described above, there is a possibility that the detection switch may detect "liquid supply completion (zero residual liquid)" even though the liquid chemical remains in the liquid chemical container. As a result, the liquid chemical administration device can no longer administer an amount of the liquid chemical that should be originally administered to the living body.

The present invention has been made in view of the above problem, and an object of the present invention is to provide a liquid chemical administration device and a control method for a liquid chemical administration device that can prevent liquid supply of a liquid chemical from ending in a state where a liquid chemical remains in a liquid chemical container.

### Solution to Problem

The liquid chemical administration device of the present invention has: a liquid chemical container filled with a liquid chemical and having a distal end opening at a distal end; a housing that holds the liquid chemical container; a pusher that pushes out the liquid chemical in the liquid chemical container; a drive mechanism that advances the pusher toward the distal end opening of the liquid chemical container; a detection unit that detects a detection target part of the pusher and detects liquid supply completion of the liquid chemical based on a detection result; and a control unit that controls an operation of the drive mechanism. The control unit further advances the pusher after the detection unit detects the liquid supply completion.

Further, a control method for a liquid chemical administration device of the present invention is a control method for controlling movement of a pusher in a liquid chemical administration device including: a liquid chemical container filled with a liquid chemical and having a distal end opening at a distal end; a housing that holds the liquid chemical container; the pusher that pushes out the liquid chemical in the liquid chemical container; a drive mechanism that advances the pusher toward the distal end opening of the liquid chemical container; a detection unit that detects a detection target part of the pusher and detects liquid supply completion of the liquid chemical based on a detection result; and a control unit that controls an operation of the drive mechanism. The control unit further advances the pusher after the detection unit detects the liquid supply completion.

### Advantageous Effects of Invention

According to the liquid chemical administration device and the control method for the liquid chemical administration device of the present invention, it is possible to prevent liquid supply of the liquid chemical from ending in a state where the liquid chemical remains in the liquid chemical container.

### Brief Description of Drawings

Fig. 1 is a side view of a liquid chemical administration system according to an embodiment.
Fig. 2 is a view schematically showing a usage example of the liquid chemical administration system.
Fig. 3 is a schematic perspective view of a liquid chemical administration device.
Fig. 4 is a schematic perspective view of a chassis included in a housing, and each component member assembled to the chassis.
Fig. 5 is a block diagram schematically showing a control system of the liquid chemical administration device.
Fig. 6 is a flowchart schematically showing a control method for a liquid chemical administration device according to the embodiment.
Fig. 7 is a plan view of the liquid chemical administration device, showing a state before a pusher moves forward.
Fig. 8 is a plan view of the liquid chemical administration device, showing a state after the pusher moves forward.
Fig. 9 is a view showing a positional relationship between the pusher and a detection unit.
Fig. 10 is a plan view showing a contrast example.
Fig. 11 is a plan view showing a contrast example.

### Description of Embodiments

Hereinafter, an embodiment of the present invention will be described with reference to the accompanying drawings. Note that the following description does not limit the technical scope or the meaning of terms used in the claims. Further, the dimensional ratios in the drawings are exaggerated for convenience of description, and may differ from the actual ratios.

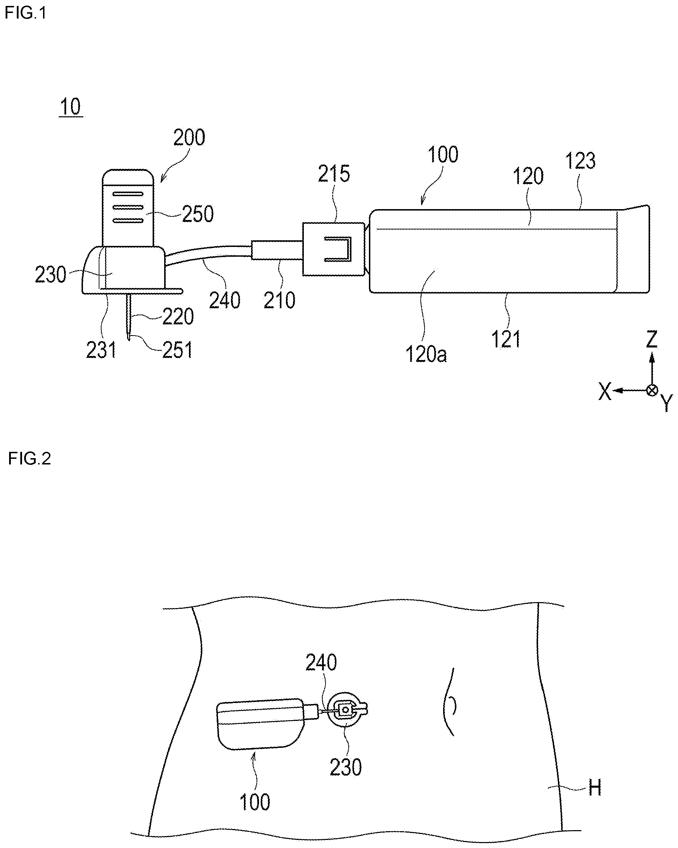

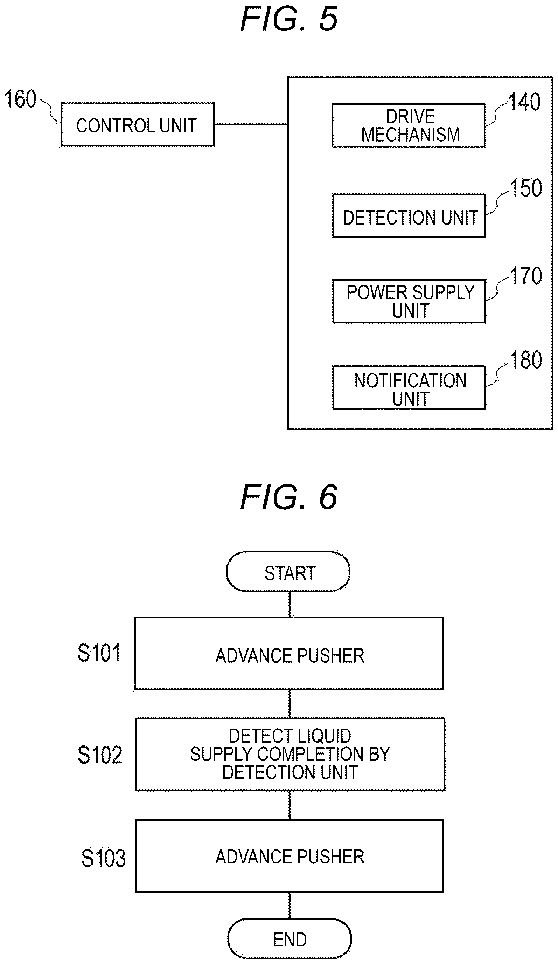

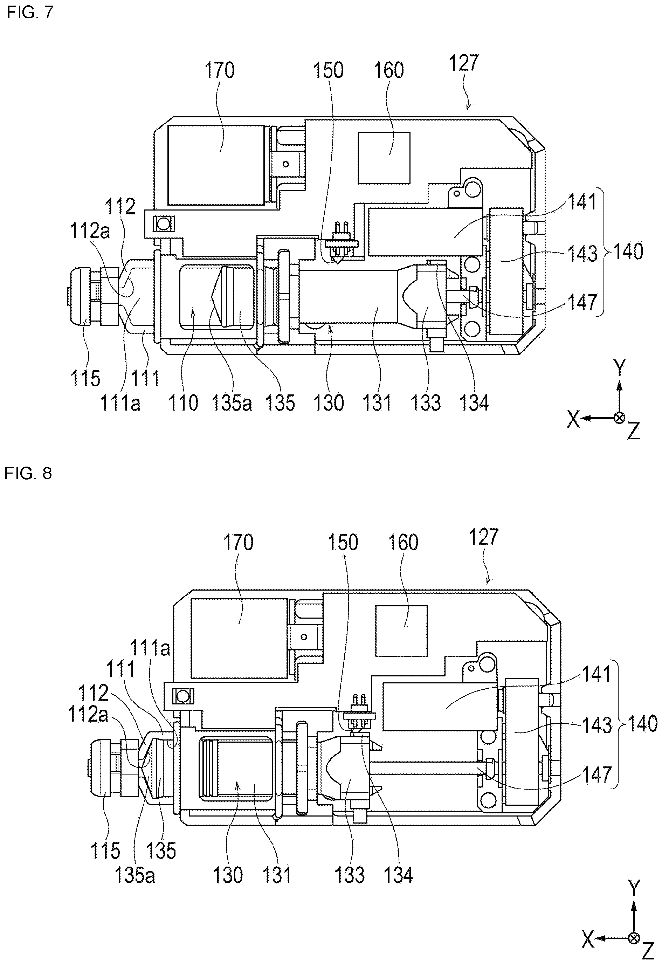

Figs. 1 to 4 are views provided for explaining a liquid chemical administration system 10, a liquid chemical administration device 100, and an administration tool 200 according to the embodiment. Fig. 5 is a block diagram schematically showing a control system of a control unit 160, and Fig. 6 is a flowchart showing a control method for a liquid chemical administration device. Further, Figs. 7 to 8 are views for explaining an action of the liquid chemical administration device 100 and a comparative example. Arrow X attached to each figure indicates a "longitudinal direction (a longitudinal direction of a liquid chemical container 110)" of the liquid chemical administration device 100, arrow Y indicates a "width direction (a depth direction)" of the liquid chemical administration device 100, and arrow Z indicates a "height direction" of the liquid chemical administration device 100.

### (Liquid chemical administration system)

The liquid chemical administration system 10 is used to administer a liquid chemical into a living body. As shown in Fig. 1, the liquid chemical administration system 10 includes the liquid chemical administration device 100 and the administration tool 200.

As shown in Fig. 2, the liquid chemical administration device 100 and the administration tool 200 are configured as a patch type that is used by being attached to a body surface (skin) H of a user. A body part of the user to be attached with the liquid chemical administration device 100 and the administration tool 200 is not particularly limited, but is, for example, an abdomen or a thigh.

The liquid chemical administration system 10 can continuously administer, for example, a liquid chemical (not shown) filled in the liquid chemical container 110 included in the liquid chemical administration device 100, into a living body over a relatively long time (for example, several minutes to several hours) through a pushing action of a pusher 130 described later. Note that the liquid chemical administration system 10 may intermittently administer the liquid chemical into the living body.

### (Liquid chemical administration device)

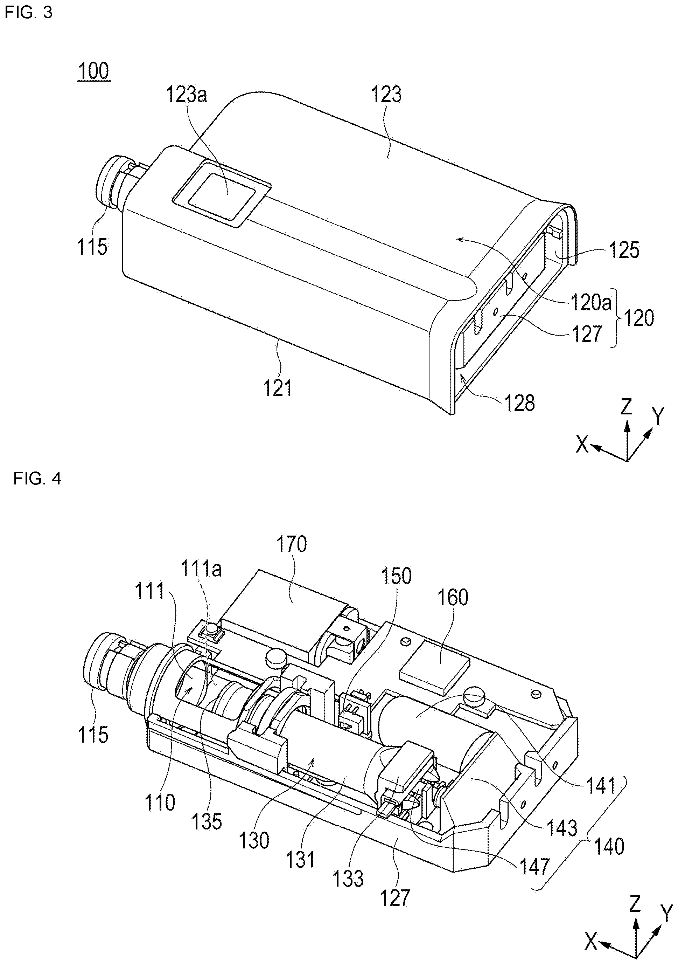

As shown in Figs. 3, 4, 7, and 8, the liquid chemical administration device 100 has the liquid chemical container 110 including a cylindrical (barrel-shaped) main body 111 filled with a liquid chemical, a housing 120 that holds the liquid chemical container 110, the pusher 130 that pushes out the liquid chemical in the liquid chemical container 110, a drive mechanism 140 that advances the pusher 130 toward a distal end opening of the liquid chemical container 110, a detection unit 150 that detects a detection target part 134 of the pusher 130 and detects liquid supply completion of the liquid chemical based on a detection result, and the control unit 160 that controls an operation of the drive mechanism.

As shown in Figs. 3 and 4, the housing 120 has a box-shaped housing main body 120a in which an accommodation space 128 is internally formed, and a chassis (corresponding to a "supporting part") 127 that is accommodated in the accommodation space 128 of the housing main body 120a and can be fixed to the housing main body 120a.

As shown in Fig. 3, on an upper surface 123 of the housing main body 120a, there is formed a window 123a that enables visual recognition of an inside of the accommodation space 128 from an outside of the housing 120. The window 123a is formed by providing a transparent or semitransparent portion on a part of the housing main body 120a.

On a base end side in the longitudinal direction of the housing main body 120a, there is formed a base end opening 125 for insertion of the chassis 127 into the accommodation space 128 of the housing main body 120a. The base end opening 125 of the housing main body 120a is closed by a lid member (not shown), with the chassis 127 accommodated in the accommodation space 128.

A bottom surface 121 of the housing main body 120a is provided with a sheet-shaped sticking part (not shown) that can be stuck to the body surface H of the user. In an initial state before attaching the liquid chemical administration device 100 to the user, a releasable protective sheet is attached to a sticking surface of the sticking part.

As shown in Fig. 4, the chassis 127 holds the liquid chemical container 110, the pusher 130, the drive mechanism 140, the detection unit 150, the control unit 160, and a power supply unit 170.

The liquid chemical container 110 is configured by a so-called pre-filled liquid chemical container. Therefore, the liquid chemical is filled in advance in an inner cavity 111a of the main body 111 of the liquid chemical container 110. Examples of the liquid chemical include a protein preparation, a narcotic analgesic, a diuretic, and the like.

At a distal end opening (a discharge port) formed at a distal end 112 of the liquid chemical container 110, a sealing member (not shown) to prevent leaking of the liquid chemical is arranged. As shown in Fig. 3, the distal end opening of the liquid chemical container 110 is arranged so as to externally project from the housing main body 120a. Further, a mounting part 115, which is to be connected to a tube 240 (see Fig. 1) described later, is attached to a distal end portion of the liquid chemical container 110 projecting from the housing main body 120a.

A main body 131 of the pusher 130 is inserted into the inner cavity 111a of the main body 111 of the liquid chemical container 110 (see Figs. 4 and 7). At a distal end of the main body 131 of the pusher 130, a gasket 135 that is slidable with an inner wall of the liquid chemical container 110 is arranged. In the gasket 135, a base end side of the gasket 135 is liquid-tightly sealed, by an outer peripheral portion of the gasket 135 being in liquid-tight contact with an inner peripheral surface of the main body 111 of the liquid chemical container 110.

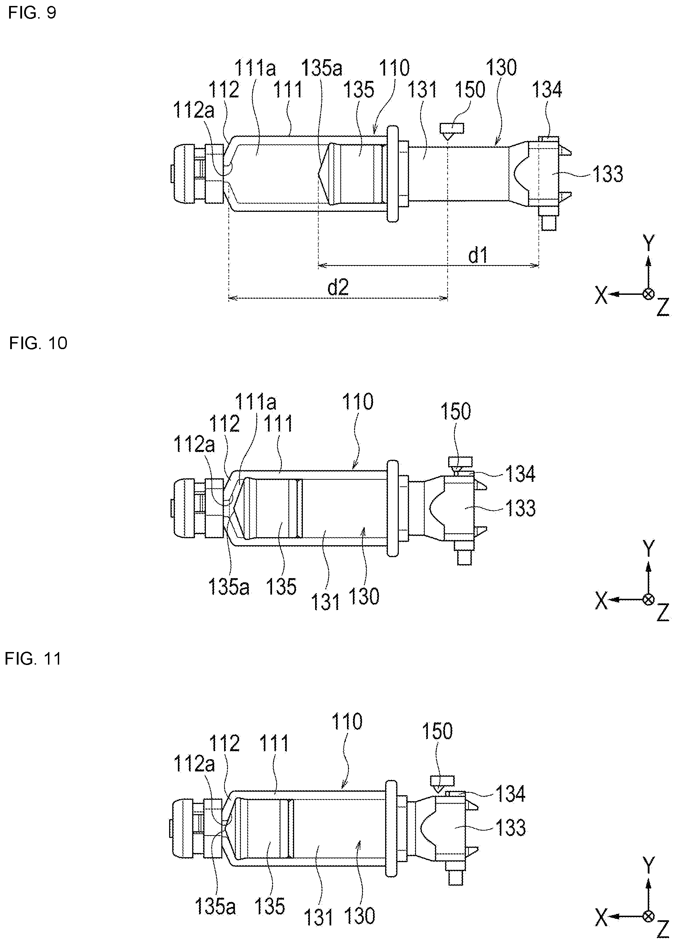

In the present embodiment, the gasket 135 is configured to be able to contract in an advancement direction (the longitudinal direction) of the pusher 130, when the pusher 130 advances in a state where the gasket 135 abuts against a distal-end inner wall 112a (see Fig. 9) of the liquid chemical container 110. The gasket 135 can be made by a flexible resin material such as, for example, a rubber material or an elastomer so as to be able to contract as described above.

As shown in Fig. 9, the gasket 135 has a tapered shape in which an outer diameter decreases toward a distal end side. Further, the shape of the gasket 135 is formed to be substantially the same as a shape of the distal-end inner wall 112a of the liquid chemical container 110.

As shown in Fig. 9, the detection target part 134 is provided at a base end of the pusher 130. The detection target part 134 is used to detect liquid supply completion of the liquid chemical by the liquid chemical administration device 100. The method of detecting the liquid supply completion will be described later.

The control unit 160 controls a liquid supply operation of the liquid chemical of the liquid chemical administration device 100. The control unit 160 can be configured by, for example, a known microcomputer (an electronic circuit element) mounted with a CPU, a RAM, a ROM, and the like. The control unit 160 centrally executes operation control of the drive mechanism 140, the detection unit 150, the power supply unit 170, and a notification unit 180 (see Fig. 5).

As shown in Fig. 7, the detection unit 150 is arranged on the chassis 127. As shown in Fig. 8, when the detection target part 134 included in the pusher 130 comes into contact with the detection unit 150, the detection unit 150 detects liquid supply completion of the liquid chemical administration device 100. The detection unit 150 can be configured by, for example, a known contact sensor that emits a predetermined electric signal when the detection target part 134 comes into contact with the detection unit 150. The control unit 160 receives the electric signal from the detection target part 134, to acquire information regarding the liquid supply completion. Note that the detection unit 150 is not particularly limited in a specific configuration and the like as long as it is possible to detect a position of the detection target part 134 of the pusher 130 when the pusher 130 advances by a predetermined amount.

The power supply unit 170 can be configured by, for example, a known battery or the like.

The notification unit 180 notifies the user of liquid supply completion. The notification unit 180 can be configured by, for example, a speaker or the like that emits voice or sound indicating that the liquid supply is completed. The notification unit 180 can be accommodated in the accommodation space 128 of the housing main body 120a.

As shown in Fig. 4, the drive mechanism 140 has a motor 141 that receives a driving current from the power supply unit 170 to generate a rotational driving force, a reduction mechanism 143 including a gear or the like to transmit the rotational driving force of the motor 141, and a feed screw 147 connected to the reduction mechanism 143.

The feed screw 147 is connected to a base-end connecting part 133 arranged near the base end of the pusher 130. The feed screw 147 converts a rotary motion transmitted from the reduction mechanism 143 into a linear motion, and advances the pusher 130 in the longitudinal direction (the X direction). The pusher 130 pushes out the liquid chemical from the inner cavity 111a of the main body 111 of the liquid chemical container 110 to the tube 240 (see Fig. 1), by advancing toward the distal end side of the liquid chemical container 110.

Next, with reference to Fig. 9, a positional relationship between the detection unit 150 and the detection target part 134 will be described. Note that Fig. 9 shows the liquid chemical container 110 and the pusher 130 in a state before administering the liquid chemical.

In the present embodiment, the detection unit 150 is arranged so as to detect liquid supply completion before a distal end 135a of the pusher 130 abuts against the distal-end inner wall 112a of the liquid chemical container 110. More specifically, as shown in Fig. 9, a first distance (d1) from the distal end 135a of the pusher 130 to the detection target part 134 is shorter than a second distance (d2) from the distal-end inner wall 112a of the liquid chemical container 110 to a position where the detection unit 150 is arranged. Therefore, when the pusher 130 advances in the liquid chemical container 110 to cause the detection target part 134 to come into contact with the detection unit 150, and the detection unit 150 detects liquid supply completion, there is formed a gap (space) in which the pusher 130 can further advance, between the distal-end inner wall 112a of the liquid chemical container 110 and the distal end 135a of the pusher 130.

Note that the distal end 135a of the pusher 130 means the distal end of the gasket 135 when the gasket 135 is arranged on the pusher 130 as in the present embodiment.

Next, a control method for the liquid chemical administration device 100 according to the present embodiment will be described. Hereinafter, operation control of the pusher 130 at a time of liquid supply of a liquid chemical will be described in detail.

As shown in Fig. 6, the control method for the liquid chemical administration device 100 has a step of advancing the pusher 130 in the liquid chemical container 110 (S101), a step of detecting liquid supply completion by the detection unit 150 detecting the detection target part 134 of the pusher 130 (S102), and a step of advancing the pusher 130 after liquid supply completion is detected by the detection unit 150 (S103).

Fig. 7 shows a state before the pusher 130 advances in the liquid chemical container 110. When the pusher 130 advances in the liquid chemical container 110 from the state shown in Fig. 7, and the detection target part 134 comes into contact with the detection unit 150 as shown in Fig. 8, the detection unit 150 detects the liquid supply completion.

In the control method according to the present embodiment, after the liquid supply completion is detected as described above, the pusher 130 is further advanced by a distance equal to or more than a distance until the distal end 135a of the pusher 130 abuts against the distal-end inner wall 112a of the liquid chemical container 110. This is for the following reason.

For example, in the liquid chemical administration device 100, if there is a deviation in installation accuracy of the detection unit 150, the pusher 130, the liquid chemical container 110, and the like, or dimensional accuracy of the pusher 130, the liquid chemical container 110, and the like, as shown in Figure 10, the detection unit 150 detects liquid supply completion despite being in a state where the liquid chemical remains in the liquid chemical container 110 (a state where the distal end 135a of the pusher 130 is not in contact with the distal-end inner wall 112a of the liquid chemical container 110). If the liquid supply by the liquid chemical administration device 100 ends as it is, it becomes impossible to administer an amount of the liquid chemical that should be originally administered to the living body. With respect to such a problem, the liquid chemical administration device 100 according to the present embodiment further advances the pusher 130 after the detection unit 150 detects the liquid supply completion. Therefore, the liquid chemical administration device 100 can preferably prevent occurrence of "remaining of the liquid chemical" due to a deviation or the like in installation accuracy or dimensional accuracy of each member. Particularly, in the present embodiment, it is possible to more reliably prevent the liquid chemical from remaining in the liquid chemical container 110 since the pusher 130 is advanced by a distance equal to or more than a distance until the distal end 135a of the pusher 130 abuts against the distal-end inner wall 112a of the liquid chemical container 110 after the liquid supply completion is detected.

Further, if there is a deviation or the like in installation accuracy or dimensional accuracy of each member, as shown in Fig. 11, there may also be a problem that the detection unit 150 does not detect the liquid supply completion although the pusher 130 has advanced until the distal end 135a of the pusher 130 abuts against the distal-end inner wall 112a of the liquid chemical container 110. For such a problem, the gasket 135 included in the pusher 130 according to the present embodiment is configured to be able to contract in a state of abutting against the distal-end inner wall 112a of the liquid chemical container 110, in an advancement direction (the longitudinal direction) of the pusher 130 when the pusher 130 advances. Therefore, the liquid chemical administration device 100 can further advance the pusher 130 by an amount of contraction of the gasket 135, even if the state shown in Fig. 11 is made to cause a situation where the advancement of the pusher 130 does not proceed. Therefore, in the liquid chemical administration device 100, the detection unit 150 can reliably detect the liquid supply completion.

Note that a timing for ending the advancement of the pusher 130 and a timing for notifying the liquid supply completion may be set at any timing, for example, after a predetermined time has elapsed after the detection unit 150 detects the liquid supply completion. Further, the advancement of the pusher 130 may be continued until the liquid chemical administration device 100 is removed from the living body, or may be continued as it is after the liquid supply is completed.

### (Administration tool)

As shown in Figs. 1 and 2, the administration tool 200 is configured to be connectable to the liquid chemical administration device 100.

The administration tool 200 has a connector 210, a needle tube 220 that is punctured into a living body, a puncture part (a cannula housing) 230, the tube 240, and a puncture assisting tool 250 that assists puncture of the needle tube 220 into the living body.

The connector 210 is configured to be connectable to the liquid chemical administration device 100 via a mounting part 215 fixed to the connector 210. The mounting part 215 can be connected to the liquid chemical administration device 100 by being externally fitted to the mounting part 115 (see Fig. 4) provided near the distal end 112 of the liquid chemical container 110 projecting to an outside of the housing 120.

Inside the mounting part 215, there is arranged a connecting needle part (not shown) capable of puncturing a sealing member (not shown) arranged at the distal end portion of the liquid chemical container 110. The tube 240 communicates with the inner cavity 111a of the main body 111 of the liquid chemical container 110 via the connecting needle part.

Inside the puncture part 230, a flow path (not shown) that connects the tube 240 and an inner cavity of the needle tube 220 is formed. A liquid chemical supplied to the puncture part 230 via the tube 240 is administered into the living body through the flow path formed inside the puncture part 230 and the needle tube 220.

The puncture assisting tool 250 is attached to the puncture part 230 at a time of liquid supply of the liquid chemical to the user. The puncture assisting tool 250 holds an introduction needle (an inner needle) 251. The introduction needle 251 projects from a distal end of the needle tube 220, with the puncture assisting tool 250 attached to the puncture part 230. By puncturing the needle tube 220 into the living body with the introduction needle 251 inserted through the needle tube 220, the user can insert the needle tube 220 into a living body while preventing breakage or the like of the needle tube 220.

The puncture assisting tool 250 is removed from the puncture part 230 after the needle tube 220 is punctured into the living body. The introduction needle 251 is extracted from the inner cavity of the needle tube 220 when the puncture assisting tool 250 is removed from the puncture part 230.

After the needle tube 220 is punctured into the living body, the puncture assisting tool 250 is removed, and the puncture part 230 is left on the body surface H of the user with the needle tube 220 placed in the living body. In this state, as the pusher 130 of the liquid chemical administration device 100 advances in the liquid chemical container 110, the liquid chemical filled in the liquid chemical container 110 is supplied to the inner cavity of the needle tube 220 via the tube 240 and the flow path of the puncture part 230.

The introduction needle 251 can be configured by, for example, a metal needle. Further, the needle tube 220 can be configured by, for example, a resin tubular member (a cannula).

The administration tool 200 is configured as a patch type to be used by being attached to the body surface H of the user, similarly to the liquid chemical administration device 100. A contact surface (a bottom surface) 231 of the puncture part 230 of the administration tool 200 is provided with a sheet-shaped sticking part (not shown) that can be stuck to the body surface. In an initial state before attaching the administration tool 200 to the user, a releasable protective sheet is attached to a sticking surface of the sticking part.

As described above, the liquid chemical administration device 100 according to the present embodiment has the liquid chemical container 110 filled with a liquid chemical and having the distal end opening at the distal end 112, the housing 120 that holds the liquid chemical container 110, the pusher 130 that pushes out the liquid chemical in the liquid chemical container 110, the drive mechanism 140 that advances the pusher 130 toward the distal end opening of the liquid chemical container 110, the detection unit 150 that detects the detection target part 134 of the pusher 130 and detects liquid supply completion of the liquid chemical based on a detection result, and the control unit 160 that controls an operation of the drive mechanism 140. The control unit 160 further advances the pusher 130 after the detection unit 150 detects the liquid supply completion.

According to the liquid chemical administration device 100 configured as described above, it is possible to prevent advancement of the pusher 130 from ending in a state where the liquid chemical remains in the liquid chemical container 110, since the pusher 130 is further advanced after liquid supply completion is detected by the detection unit 150. Therefore, it is possible to prevent liquid supply of the liquid chemical from ending in a state where the liquid chemical remains in the liquid chemical container 110.

Further, in the liquid chemical administration device 100, the detection unit 150 is arranged so as to detect liquid supply completion before the distal end 135a of the pusher 130 abuts against the distal-end inner wall 112a of the liquid chemical container 110. Furthermore, the control unit 160 advances the pusher 130 by a distance equal to or more than a distance until the distal end 135a of the pusher 130 abuts against the distal-end inner wall 112a of the liquid chemical container 110 after the detection unit 150 detects the liquid supply completion. Therefore, the liquid chemical administration device 100 can more reliably prevent liquid supply of the liquid chemical from ending in a state where the liquid chemical remains in the liquid chemical container 110.

Further, in the liquid chemical administration device 100, the first distance (d1) from the distal end 135a of the pusher 130 to the detection target part 134 is shorter than the second distance (d2) from the distal-end inner wall 112a of the liquid chemical container 110 to the position where the detection unit 150 is arranged. Therefore, the detection unit 150 can reliably detect liquid supply completion before the distal end 135a of the pusher 130 abuts against the distal-end inner wall 112a of the liquid chemical container 110. Therefore, by further advancing the pusher 130 after detecting the liquid supply completion, it is possible to more reliably prevent liquid supply of the liquid chemical from ending in a state where the liquid chemical remains in the liquid chemical container 110.

Further, in the liquid chemical administration device 100, the gasket 135 slidable with respect to the inner wall of the liquid chemical container 110 is arranged at the distal end 135a of the pusher 130. Further, when the pusher 130 advances, the gasket 135 can contract in a state of abutting against the distal-end inner wall 112a of the liquid chemical container 110, in an advancement direction of the pusher 130. Therefore, the pusher 130 can be further advanced by an amount of contraction of the gasket 135, even in a state where the gasket 135 abuts against the distal-end inner wall 112a of the liquid chemical container 110 and a state where liquid supply completion is not detected by the detection unit 150. Therefore, the liquid chemical administration device 100 can more reliably detect the liquid supply completion.

Further, the control method for the liquid chemical administration device 100 according to the present embodiment is a control method for controlling movement of the pusher 130 in the liquid chemical administration device 100 including the liquid chemical container 110 filled with a liquid chemical and having the distal end opening at the distal end 112, the housing 120 that holds the liquid chemical container 110, the pusher 130 that pushes out the liquid chemical in the liquid chemical container 110, the drive mechanism 140 that advances the pusher 130 toward the distal end opening of the liquid chemical container 110, the detection unit 150 that detects the detection target part 134 of the pusher 130 and detects liquid supply completion of the liquid chemical based on a detection result, and the control unit 160 that controls an operation of the drive mechanism 140. In the control method, the control unit 160 further advances the pusher 130 after the detection unit 150 detects the liquid supply completion.

According to the above control method, since the pusher 130 is further advanced after liquid supply completion is detected by the detection unit 150, it is possible to prevent advancement of the pusher 130 from ending in a state where the liquid chemical remains in the liquid chemical container 110. Therefore, it is possible to prevent liquid supply of the liquid chemical from ending in a state where the liquid chemical remains in the liquid chemical container 110.

### <Modified example>

The liquid chemical administration device 100 can also be configured to determine an administration state, for example, on the basis of a detection result of a rotation state of the motor 141 and on the basis of detection of liquid supply completion based on contact between the detection unit 150 and the detection target part 134 of the pusher 130. Specifically, the control unit 160 can be configured to determine the administration state of the liquid chemical administration device 100 with the following control flow.

When rotation stop of the motor 141 is detected by a rotation detection mechanism (not shown), the control unit 160 confirms detection of liquid supply completion based on contact between the detection target part 134 of the pusher 130 and the detection unit 150. Note that, when the motor 141 is stopped, the distal end 135a of the pusher 130 is in a state of abutting against the distal-end inner wall 112a of the liquid chemical container 110 (the state shown in Fig. 11). Therefore, after the detection unit 150 detects the liquid supply completion, the control unit 160 advances the pusher 130 until the rotation detection mechanism detects a stop of the rotation of the motor 141. Then, when the motor 141 is stopped, the control unit 160 determines that administration of the liquid chemical has ended normally, when confirming the liquid supply completion based on contact between the detection target part 134 of the pusher 130 and the detection unit 150 (when the pusher 130 and the detection unit 150 are in a contact state). Whereas, when the motor 141 is stopped, the control unit 160 determines that administration of the liquid chemical has not ended normally (that is, the administration of the liquid chemical has ended with the residual liquid remaining in the liquid chemical container 110), when confirming no detection of liquid supply completion based on contact between the detection target part 134 of the pusher 130 and the detection unit 150 (when the pusher 130 and the detection unit 150 are in a noncontact state). When the control unit 160 confirms that the administration of the liquid chemical has not been normally performed, the control unit 160 can notify that effect by the notification unit 180.

As described above, the liquid chemical administration device and the control method for the liquid chemical administration device according to the present invention have been described through the embodiment. However, the present invention is not limited to each configuration described above, but can be modified as appropriate based on the description of the claims.

For example, for the control unit, a specific control method, control procedure, and the like are not particularly limited as long as an operation is controlled to further advance the pusher after the detection unit detects liquid supply completion. Further, the housing may be configured such that each component member is fixed or held in the housing main body without including the chassis.

Further, a material, a shape, a size, arrangement, a connection/coupling structure between members, and the like of each member included in the liquid chemical administration device can be freely changed and replaced without limitation as long as the effects of the present invention are exhibited. Further, it is possible to appropriately add any component members and the like not particularly described in the specification, to the liquid chemical administration device.

This application is based on Japanese Patent Application 2018-067431, filed on March 30, 2018, the entire disclosure content of which is incorporated by reference.

### Reference Signs List

10 Liquid chemical administration system,
100 liquid chemical administration device,
110 liquid chemical container,
111 main body of liquid chemical container,
111a inner cavity of liquid chemical container,
112a distal-end inner wall of liquid chemical container,
120 housing,
120a housing main body,
127 chassis,
128 accommodation space,
130 pusher,
131 main body of pusher,
134 detection target part,
135 gasket,
135a distal end of pusher,
140 drive mechanism,
150 detection unit,
160 control unit,
170 power supply unit,
180 notification unit,
200 administration tool,
H body surface.

## Claims

1. A liquid chemical administration device comprising:
a liquid chemical container filled with a liquid chemical and having a distal end opening at a distal end;
a housing that holds the liquid chemical container;
a pusher that pushes out the liquid chemical in the liquid chemical container;
a drive mechanism that advances the pusher toward the distal end opening of the liquid chemical container;
a detection unit that detects a detection target part of the pusher and detects liquid supply completion of the liquid chemical based on a detection result; and
a control unit that controls an operation of the drive mechanism, wherein
the control unit further advances the pusher after the detection unit detects the liquid supply completion.

2. The liquid chemical administration device according to claim 1, wherein
the detection unit is arranged to detect the liquid supply completion before a distal end of the pusher abuts against a distal-end inner wall of the liquid chemical container, and
after the detection unit detects the liquid supply completion, the control unit advances the pusher by a distance equal to or more than a distance until the distal end of the pusher abuts against the distal-end inner wall of the liquid chemical container.

3. The liquid chemical administration device according to claim 2, wherein a first distance from the distal end of the pusher to the detection target part is shorter than a second distance from the distal-end inner wall of the liquid chemical container to a position where the detection unit is arranged.

4. The liquid chemical administration device according to any one of claims 1 to 3, wherein
a gasket slidable with respect to an inner wall of the liquid chemical container is arranged at the distal end of the pusher, and
when the pusher advances, the gasket can contract in a state of abutting against the distal-end inner wall of the liquid chemical container, in an advancement direction of the pusher.

5. A control method for a liquid chemical administration device, the control method being for controlling movement of a pusher in a liquid chemical administration device comprising: a liquid chemical container filled with a liquid chemical and having a distal end opening at a distal end; a housing that holds the liquid chemical container; the pusher that pushes out the liquid chemical in the liquid chemical container; a drive mechanism that advances the pusher toward the distal end opening of the liquid chemical container; a detection unit that detects a detection target part of the pusher and detects liquid supply completion of the liquid chemical based on a detection result; and a control unit that controls an operation of the drive mechanism, wherein
the control unit further advances the pusher after the detection unit detects the liquid supply completion.
